# FASCICULE DE BREVET EUROPEEN

(11) **EP 1 515 687 B1**
(45) Date de publication et mention de la délivrance du brevet: **20.11.2013**
(21) Numéro de dépôt: 03757133.8
(22) Date de dépôt: 10.06.2003
(51) Int. Cl.: A61K 8/34, A61K 8/42, A61K 8/63, A61K 8/64, A61K 31/7048, A61Q 5/00, A61Q 5/10, A61Q 7/00, A61Q 5/06

(54) **Utilisation d'un agent inducteur de l'expression de la dopachrome tautomerase (TRP-2) comme agent protecteur des mélanocytes du follicule pileux**
Verwendung eines Wirkstoffs, der die Expression der Dopachrome Tautomerase (TRP-2) induziert, als Schutzmittel für Haarfollikel-Melanozyten
Use of an agent inducing dopachrome tautomerase (TRP-2) expression as protecting agent for hair follicle melanocytes

(30) Priorité: 11.06.2002 FR 0207136
(43) Date de publication de la demande: 23.03.2005
(73) Titulaire: L'Oréal, 75008 Paris (FR)
(72) Inventeur: COMMO, Stéphane, F-75015 Paris (FR); BERNARD, Bruno, F-92200 Neuilly sur Seine (FR)
(74) Mandataire: Allab, Myriam
(86) Numéro de dépôt international: PCT/FR2003/001728
(87) Numéro de publication internationale: WO 2003/103568

(56) Documents cités:
- EP-A- 0 124 077
- EP-A- 0 327 345
- EP-A- 0 580 409
- WO-A-02/30371
- WO-A-98/11882
- FR-A- 2 691 465
- US-A- 5 965 157
- US-A- 6 132 980
- DATABASE WPI Week 198639 Derwent Publications Ltd., London, GB; AN 1986-255367 XP002234442 & JP 61 183207 A (KANEBO) 15 août 1986 (1986-08-15)
- PATENT ABSTRACTS OF JAPAN vol. 015, no. 238 (C-0841), 19 juin 1991 (1991-06-19) & JP 03 074318 A (KATSUMI MIZUMAKI), 28 mars 1991 (1991-03-28)
- DATABASE WPI Week 199836 Derwent Publications Ltd., London, GB; AN 1998-422294 XP002234443 & JP 10 175854 A (POLA CHEM IND) 30 juin 1998 (1998-06-30)
- DATABASE BIOSIS [Online] BIOSCIENCES INFORMATION SERVICE, PHILADELPHIA, PA, US; juin 2001 (2001-06), DUNN KAREN J ET AL: "In utero complementation of a neural crest-derived melanocyte defect using cell directed gene transfer." XP002234437 Database accession no. PREV200100385010 & GENESIS THE JOURNAL OF GENETICS AND DEVELOPMENT, vol. 30, no. 2, juin 2001 (2001-06), pages 70-76, ISSN: 1526-954X
- DATABASE BIOSIS [Online] BIOSCIENCES INFORMATION SERVICE, PHILADELPHIA, PA, US; 1995, PROTA GIUSEPPE ET AL: "Comparative analysis of melanins and melanosomes produced by various coat color mutants." XP002234438 Database accession no. PREV199598424243 & PIGMENT CELL RESEARCH, vol. 8, no. 3, 1995, pages 153-163, ISSN: 0893-5785
- DATABASE CAPLUS [Online] CHEMICAL ABSTRACTS SERVICE, COLUMBUS, OHIO, US; XP002234439 accession no. STN Database accession no. 1999:5979
- DATABASE CAPLUS [Online] CHEMICAL ABSTRACTS SERVICE, COLUMBUS, OHIO, US; XP002234440 accession no. STN Database accession no. 2001:785322
- DATABASE CAPLUS [Online] CHEMICAL ABSTRACTS SERVICE, COLUMBUS, OHIO, US; XP002234441 accession no. STN Database accession no. 1998:246143

## Description

La présente invention se rapporte à l'utilisation cosmétique d'un agent inducteur de l'expression de la DOPAchrome tautomérase, en tant qu'agent protecteur des mélanocytes du follicule pileux. En particulier, l'agent inducteur de l'expression de la DOPAchrome tautomérase est destiné à lutter contre la disparition des mélanocytes du follicule pileux en maintenant et/ou régénérant la population des mélanocytes actifs du bulbe et des mélanocytes quiescents de la région supérieure du follicule pileux.

Le follicule pileux est une invagination tubulaire de l'épiderme qui s'enfonce jusqu'aux couches profondes du derme. La partie inférieure, ou bulbe pileux, comporte elle-même une invagination dans laquelle se trouve la papille dermique. La partie inférieure du bulbe est une zone de prolifération cellulaire où se trouvent les précurseurs des cellules kératinisées constituant le cheveu. Les cellules en ascension issues de ces précurseurs se kératinisent progressivement dans la partie supérieure du bulbe, et cet ensemble de cellules kératinisées formera la tige pilaire.

La couleur des cheveux et des poils repose notamment sur la présence en quantités et ratios variables de deux groupes de mélanines : les eumélanines (pigments bruns et noirs) et les phéomélanines (pigments rouges et jaunes). La pigmentation du cheveu et des poils requiert la présence de mélanocytes au niveau du bulbe du follicule pileux. Ces mélanocytes sont dans un état actif, c'est-à-dire qu'ils synthétisent des mélanines. Ces pigments sont transmis aux kératinocytes destinés à former la tige pilaire ce qui conduira à la pousse d'un cheveu ou d'un poil pigmenté. Cette structure est appelée ci-après « unité folliculaire de pigmentation ».

Chez les mammifères, la mélanogénèse implique au moins trois enzymes : la tyrosinase, la DOPAchrome tautomérase (TRP-2, pour Tyrosinase Related Protein 2) et la DHICAoxydase (TRP-1, pour Tyrosinase Related Protein 1).

La tyrosinase est l'enzyme qui initie la biosynthèse des mélanines. Elle est également décrite comme étant l'enzyme limitant de la mélanogénèse.

La TRP-2 catalyse la tautomérisation du DOPAchrome en acide 5,6-Dihydroxyindole-2-carboxylique (DHICA). En l'absence de TRP-2, le DOPAchrome subit une décarboxylation spontanée pour former le 5,6-dihydroxyindole (DHI).

DHICA et DHI sont tous deux des précurseurs de pigments, TRP-1 oxyde les molécules de DHICA pour former des dérivés de quinones (Pawelek JM and Chakraborty AK. The enzymology of melanogenesis. In: Nordlund JJ, Boissy RE, Hearing VJ, King RA, Ortonne J-P. The Pigmentary System: Physiology and Pathophysiology. New York: Oxford university press; 1998. p. 391-400).

Les trois enzymes, tyrosinase, TRP-2 et TRP-1, apparaissent spécifiquement impliquées dans la mélanogénèse. De plus, l'activité de ces trois enzymes a été décrite comme nécessaire à l'activité maximale de biosynthèse des eumélanines.

L'expression de TRP-2 a été observée dans les poils de souris noires, à la fois dans les mélanocytes actifs du bulbe et dans les mélanocytes quiescents de la gaine épithéliale externe. De plus, il est connu que l'activité DOPAchrome tautomérase est augmentée pendant la phase anagène chez la souris noire. Cependant aucune corrélation claire n'a été établie entre l'expression de TRP-2 et l'intensité de la pigmentation (Sturm et al. 1995).

Par ailleurs, TRP-2 a également été décrite comme conférant aux mélanocytes qui l'expriment une résistance à des agents endommageant l'ADN tel que le cis-diamminedichloroplatinum(II) (Chu et al. 2000 et Pak et al. 2000). Ces résultats suggèrent que TRP-2 serait aussi impliquée dans une fonction indépendante de la mélanogènèse, l'enzyme pourrait jouer un rôle cytoprotecteur.

Le cheveu et le poil subissent un cycle. Ce cycle comprend une phase de croissance (phase anagène), une phase de dégénérescence (phase catagène) et une phase de repos (phase télogène) à la suite de laquelle une nouvelle phase anagène se développera. En raison de ce cycle pilaire, et contrairement à l'unité de pigmentation épidermique, l'unité folliculaire de pigmentation doit également être cycliquement renouvelée.

Ce processus a été récemment décrit chez l'homme (Commo S. et Bernard B., 2000, Pigment Cell Res. 13:253-259). Il a plus particulièrement été montré qu'au cours de la transition télogène-anagène, une partie des mélanocytes inactifs contenus dans la capsule télogène prolifère, se positionne autour de la papille dermique du bulbe naissant et commence à exprimer des enzymes nécessaires à la synthèse de mélanines : cette population de mélanocytes correspond aux mélanocytes actifs du bulbe. En parallèle, l'autre partie des mélanocytes reste inactive dans la région supérieure du follicule pileux : cette population de mélanocytes correspond aux mélanocytes quiescents de la région supérieure du follicule pileux.

Ces enzymes mélanogènes seront exprimées dans les mélanocytes du bulbe pendant toute la durée de la phase anagène mais ne seront plus exprimées pendant les phases catagène et télogène. Le cycle normal des mélanocytes dans le follicule pileux humain requiert la présence de mélanocytes quiescents dans la région supérieure du follicule. pileux, région autrement nommée « réservoir », qui seront cycliquement activés pour régénérer l'unité folliculaire de pigmentation. Ce mécanisme de renouvellement cellulaire participant au maintien de la pigmentation est spécifique de l'unité folliculaire de pigmentation ; on ne le retrouve pas dans l'unité épidermique de pigmentation.

Il est admis que la canitie (blanchissement naturel des cheveux) est associée à une diminution de mélanine dans la tige pilaire. La cause de cette diminution n'est pas à ce jour élucidée. Plusieurs hypothèses sont avancées, elle pourrait être liée à une diminution de l'activité mélanogène, par analogie au mécanisme de pigmentation de la peau, mais également à une altération du transfert des mélanines ou une à diminution du nombre de mélanocytes dans le bulbe (Tobin et Paus, 2001) ; et aucune démonstration en pigmentation cheveu n'a permis à ce jour de valider l'une ou l'autre de ces hypothèses.

Or la Demanderesse vient de mettre en évidence deux résultats qui valident pour la première fois l'hypothèse selon laquelle la canitie serait liée à une diminution du nombre de mélanocytes actifs dans le bulbe et une diminution du nombre de mélanocytes quiescents dans la région supérieure du follicule pileux. Cette diminution et/ou disparition précoce des mélanocytes est spécifique au follicule pileux et n'affecte pas de façon visible l'épiderme.

Jusqu'à présent, on pensait en effet que des mélanocytes quiescents étaient présents dans les follicules pileux de cheveux blancs (Takada et al. 1992, Horikawa et al. 1996, Jenner et Randall 2000).

Or, la Demanderesse a constaté que la progression de la canitie est associée à une diminution du nombre de mélanocytes dans les bulbes pileux, qui, bien qu'en nombre restreint, synthétisent et transfèrent les mélanines. La Demanderesse a également observé de façon inattendue et surprenante que la population de mélanocytes quiescents de la région supérieure du follicule pileux humain (encore appelée « réservoir ») est également diminuée au cours du processus de canitie, les cheveux blancs ne possédant plus que quelques - voire plus aucun - mélanocytes, contrairement à l'infundibulum et l'épiderme avoisinant ces cheveux blancs. Cette disparition affecte prématurément et spécifiquement les mélanocytes contenus dans les cheveux.

Il apparaît donc nécessaire de lutter contre la disparition des mélanocytes des follicules pileux humains, processus affectant à la fois les mélanocytes actifs des bulbes et les mélanocytes quiescents de la région supérieure des follicules pileux, pour lutter contre la canitie.

Par ailleurs, la Demanderesse a également constaté de façon inattendue que l'enzyme TRP-2 n'est pas exprimée dans les mélanocytes des follicules pileux humains pigmentés (bruns, noirs et roux) chez l'individu caucasien, asiatique et africain. Cette enzyme n'est détectée ni dans les mélanocytes actifs du bulbe, ni dans les mélanocytes quiescents de la région supérieure du follicule pileux humain alors qu'elle est exprimée dans l'épiderme et l'infundibulum de l'individu caucasien, africain et asiatique. L'absence de TRP-2 est associée à la disparition précoce des mélanocytes qui ne l'expriment pas, c'est-à-dire, les mélanocytes quiescents de la région supérieure du follicule pileux et les mélanocytes actifs du bulbe.

La Demanderesse a donc mis en évidence que TRP2, qui joue un rôle dans la mélanogénèse (synthèse de mélanine) au niveau de l'unité épidermique de pigmentation, pourrait jouer un rôle différent et méconnu jusqu'ici, dans l'unité folliculaire de pigmentation : son induction pourrait permettre de maintenir et/ou régénérer la population de mélanocytes quiescents de la région supérieure du follicule pileux et la population de mélanocytes actifs du bulbe et favoriser ainsi le renouvellement cyclique de l'unité folliculaire garant du maintien de la pigmentation des cheveux, cils et/ou poils.

La Demanderesse a montré qu'il était possible d'induire la synthèse de TRP-2. En induisant la synthèse de TRP-2, elle a identifié un moyen de maintenir et/ou régénérer la population de mélanocytes du follicule pileux responsables de la pigmentation des cheveux. Par ailleurs, elle a évalué l'activité cytoprotectrice d'agents inducteurs de TRP2 dans des conditions induisant l'apoptose et/ou la sénescence des mélanocytes du follicule pileux.

Elle a ainsi identifié un moyen pour prévenir et/ou limiter et/ou stopper le développement de la canitie et de maintenir la pigmentation des cheveux et/ou des poils gris ou blancs.

Ainsi un premier objet de l'invention se rapporte à l'utilisation cosmétique non-thérapeutique d'un agent inducteur de l'expression de la DOPAchrome tautomérase, en tant qu'agent protecteur des mélanocytes du follicule pileux.

Par 'agent protecteur des mélanocytes du follicule pileux', on entend un agent capable de protéger les mélanocytes notamment contre des agents cytotoxiques responsables de la sénescence et/ou de l'apoptose des mélanocytes du follicule pileux. Parmi les agents cytotoxiques, on peut citer des molécules à caractères génotoxiques et des molécules induisant un stress oxydatif comme le TNF alpha, les lipofuscines, le TGF beta, le ligand de Fas/CD95, L'IL1 beta, les ions ferreux et cuivreux, des composés chimiques génotoxiques comme le cisplatine et l'oxaloplatine, ou encore des composés comme le cyclophosphamide.

En particulier, l'agent inducteur de l'expression de la DOPAchrome tautomérase selon l'invention est destiné à lutter contre la disparition des mélanocytes du follicule pileux en maintenant et/ou en régénérant la population des mélanocytes actifs du bulbe et de mélanocytes quiescents de la région supérieure du follicule pileux.

L'agent inducteur de l'expression de la DOPAchrome tautomérase selon l'invention est également destiné à favoriser le renouvellement cyclique de l'unité folliculaire de pigmentation.

L'invention concerne donc l'utilisation d'un agent inducteur de l'expression de la DOPAchrome tautomérase pour prévenir et/ou limiter et/ou arrêter le développement de la canitie.

Elle porte également sur l'utilisation d'un agent inducteur de l'expression de la DOPAchrome tautomérase pour maintenir la pigmentation naturelle des cheveux et/ou des poils gris.

On entend par agent inducteur de l'expression de la DOPAchrome tautomerase un composé capable de stimuler la synthèse de l'enzyme DOPAchrome tautomérase.

Il pourra s'agir d'un vecteur d'expression codant pour la DOPAchrome tautomérase. Pour la construction de ce vecteur d'expression de l'enzyme, on préférera utiliser un promoteur spécifique d'un tissu, en particulier un promoteur spécifique de mélanocytes.

Parmi les agents inducteurs de l'expression de la DOPAchrome tautomerase (TRP-2) on peut citer les composés suivants :
- l'hexaméthylène bisacetamide (HMBA Fang et al. 2001) ;
- les hormones stéroïdiennes, telles que le diethylstilbestrol et/ou l'estradiol (Kippenberger et al. 1998) ;
- la glycyrrhizine (Jung et al. 2001) ;
- la forskoline ;
- le kaempferol.

Le document FR-A-2639828 divulgue l'utilisation du Kaempférol encapsulé dans les liposomes comme agent actif dans les compositions destinées à favoriser la pigmentation des cheveux.

L'agent inducteur de l'expression de la DOPAchrome tautomérase peut être un modulateur d'un facteur endogène, tel qu'un modulateur de l'expression de Sox10, capable d'activer le promoteur de la DOPAchrome tautomérase (TRP-2).

L'agent inducteur de l'expression de la DOPAchrome tautomerase pourra être un vecteur d'expression codant pour un agent inducteur de l'expression de la DOPAchrome tautomérase, tel que Sox10.

Pour la construction d'un vecteur d'expression d'un agent inducteur, on peut utiliser un promoteur spécifique d'un tissu, en particulier un promoteur spécifique de mélanocytes et/ou de kératinocytes.

L'expression de l'agent inducteur de la DOPAchrome tautomérase par le vecteur d'expression peut être elle-même inductible.

Selon l'invention l'agent inducteur de la DOPAchrome tautomérase est le Kaempférol encapsulé.

Un autre objet de l'invention est l'utilisation non-thérapeutique d'une composition selon la revendication 6.

La composition selon l'invention comprend une quantité d'agent inducteur de l'expression de la DOPAchrome tautomerase comprise entre 0,001 et 10 % en poids par volume, préférentiellement entre 0,01 et 5% en poids par volume et encore plus préférentiellement entre 0,1 et 1% en poids par volume.

La composition selon l'invention peut être administrée par voie orale ou appliquée sur la

peau (sur toute zone cutanée du corps recouverte de poils) et/ou le cuir chevelu ou les cheveux.

Par voie orale, la composition selon l'invention peut contenir le ou les agents inducteurs de l'expression de la DOPAchrome tautomérase composés actifs en solution dans un liquide alimentaire tel qu'une solution aqueuse ou hydroalcoolique, éventuellement aromatisée. Ils peuvent également être incorporés dans un excipient solide ingérable et se présenter par exemple sous forme de granulés, de pilules, de comprimés ou de dragées. Ils peuvent également être placés en solution dans un liquide alimentaire lui-même éventuellement conditionné dans des capsules ingérables.

Selon le mode d'administration, la composition de l'invention peut se présenter sous toutes les formes galéniques normalement utilisées, particulièrement en cosmétologie. Une composition préférée de l'invention est une composition cosmétique adaptée à une application topique sur le cuir chevelu et/ou la peau.

Pour une application topique, la composition utilisable selon l'invention peut être notamment sous la forme d'une solution aqueuse, hydroalcoolique ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Elle peut ainsi se présenter sous forme d'onguent, de teinture, de crème, de pommade, de poudre, de timbre, de tampon imbibé, de solution, d'émulsion ou de dispersion vésiculaire, de lotion, de gel, de spray, de suspension, de shampooing, d'aérosol ou de mousse. Elles peuvent être anhydres ou aqueuses. Elle peut également consister en des préparations solides constituant des savons ou des pains de nettoyage.

Ces compositions sont préparées selon les méthodes usuelles.

La composition utilisable selon l'invention peut en particulier être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, de masque.

La composition cosmétique selon l'invention sera préférentiellement une crème, une lotion capillaire, un shampoing ou un après-shampoing.

Les quantités des différents constituants des compositions utilisables selon l'invention sont celles classiquement utilisées dans les domaines considérés.

Lorsque la composition utilisable selon l'invention est une émulsion, la proportion de la phase grasse peut aller de 5% à 80% en poids, et de préférence de 5% à 50% en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition utilisable selon l'invention est une solution ou un gel huileux, la phase grasse peut représenter plus de 90% du poids total de la composition.

L'agent inducteur de l'expression de la DOPAchrome tautomérase est encapsulé dans un enrobage tel que des microsphères, des nanosphères, des oléosomes ou des nanocapsules.

A titre d'exemple, les microsphères pourront être préparées selon la méthode décrites dans la demande de brevet EP 0 375 520.

Les nanosphères pourront se présenter sous forme de suspension aqueuse et être préparées selon les méthodes décrites dans les demandes de brevet FR 0015686 et FR 0101438.

Les oléosomes consistent en une émulsion huile dans eau formée par des globules huileux pourvus d'un enrobage cristal liquide lamellaire dispersé dans une phase aqueuse (voir les demandes de brevet EP 0 641 557 et EP 0 705 593).

L'agent inducteur de l'expression de la DOPAchrome tautomérase pourra aussi être encapsulé dans des nanocapsules consistant en un enrobage lamellaire obtenu à partir d'un tensio-actif siliconé (voir la demande de brevet EP 0 780 115), les nanocapsules pourront également être préparées à base de polyesters sulfonique hydrodispersibles (voir la demande de brevet FR 0113337).

L'agent inducteur de l'expression de la DOPAchrome tautomérase pourra également être complexé à la surface de globules huileux cationiques, quelques soit leur taille (voir les demandes de brevet EP 1 010 413, EP 1 010 414, EP 1 010 415, EP 1 010 416, EP 1 013 338, EP 1 016 453, EP 1 018 363, EP 1 020 219, EP 1 025 898, EP 1 120 101, EP 1 120 102, EP 1 129 684, EP 1 160 005 et EP 1 172 077).

L'agent inducteur de l'expression de la DOPAchrome tautomérase peut enfin être complexé à la surface de nanocapsules ou nanoparticules pourvues d'un enrobage lamellaire (Voir EP 0 447 318 et EP 0 557 489) et contenant un tensio-actif cationique à la surface (voir les références citées précédemment pour les tensio-actifs cationiques).

En particulier, on préférera une composition telle que l'enrobage dans lequel l'agent inducteur de l'expression de la DOPAchrome tautomérase a un diamètre inférieur ou égal à 10 µm.

De façon connue, la composition selon l'invention peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{®} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

Les compositions utilisables selon l'invention peuvent associer au moins un agent inducteur de l'expression de TRP-2 à d'autres agents actifs. Parmi ces agents actifs, on peut citer à titre d'exemple :
- les agents modulant la différenciation et/ou la prolifération et/ou la pigmentation des cellules de la peau tels que le rétinol et ses esters, la vitamine D et ses dérivés, les oestrogènes tels que l'oestradiol, les modulateurs de l'AMPc tels que les dérivés de POMC, l'adénosine, ou la forskoline et ses dérivés, les prostaglandines et leurs dérivés, la triiodotrionine et ses dérivés ;
- des extraits de végétaux tels que ceux d'Iridacées ou de soja, extraits pouvant alors contenir ou non des isoflavones ;
- des extraits de micro-organismes ;
- les agents anti-radicaux libres tels que l'α-tocophérol ou ses esters, les superoxyde dismutases ou ses mimétiques, certains chélatants de métaux ou l'acide ascorbique et ses esters ;
- les anti-séborrhéiques tels que certains acides aminés soufrés, l'acide 13-cis rétinoïque, l'acétate de cyprotérone ;
- les autres agents de lutte contre les états desquamatifs du cuir chevelu comme le zinc pyrithione, le disulfure de sélénium, le climbazole, l'acide undécylénique, le Kétoconazole, la piroctone olamine (octopirox) ou la ciclopiroctone (ciclopirox) ;
   en particulier, il pourra s'agir d'actifs stimulant la repousse et/ou favorisant le ralentissement de la chute des cheveux, on peut plus particulièrement citer à titre non limitatif :
- les esters d'acide nicotinique, dont notamment le nicotinate de tocophérol, le nicotinate de benzyle et les nicotinates d'alkyles en C₁-C₆ comme les nicotinates de méthyle ou d'hexyle ;
- les dérivés de pyrimidine, comme le 2,4-diamino 6-piperidinopyrimidine 3-oxyde ou "Minoxidil" décrit dans les brevets US 4,139,619 et US 4,596,812 ; l'Aminexil ou 2,4 diamino pyrimidine 3 oxyde décrit dans WO96/09048 ;
- les agents inhibiteurs de lipoxygenase ou inducteur de cyclo-oxydase favorisant la repousse des cheveux comme ceux décrits par la Demanderesse dans la demande de brevet européen EP 0 648 488 ;
- les agents antibactériens tels que les macrolides, les pyranosides et les tétracyclines, et notamment l'Erythromycine ;
- les agents antagonistes de calcium, comme la Cinnarizine, la Nimodipine et la Nifedipine ;
- des hormones, telles que l'estriol ou des analogues, ou la thyroxine et ses sels ;
- des agents antiandrogènes, tels que l'oxendolone, la spironolactone et la flutamide ;
- des inhibiteurs stéroïdiens ou non stéroïdiens des 5-α-réductases tels que ceux décrits par la Demanderesse dans les demandes de brevet européen EP 0 964 852 et EP 1 068 858 ou encore le finastéride ;des agonistes des canaux potassiques dépendant de l'ATP tels que la cromakalim et le nicorandil ;
- des extraits végétaux à activité pro-pigmentante comme les extraits de chrysanthème tels que décrits dans FR 2768343 et les extraits de Sanguisorba décrits FR 2782920A1.

Les zones à traiter peuvent être, par exemple et sans aucune limitation, le cuir chevelu, les sourcils, la moustache et/ou la barbe et toute zone de la peau recouverte de poils.

### Figures :

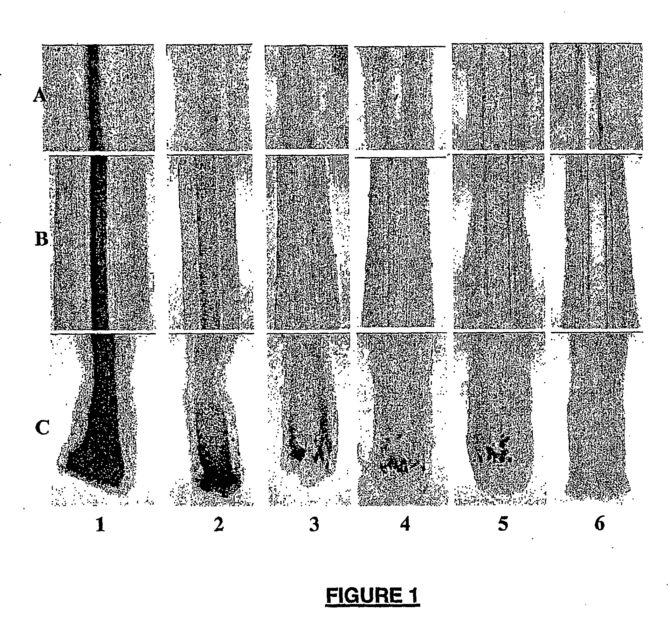

**Figure 1** **:** cette figure rassemble différentes photographies représentant la distribution de mélanocytes dans le follicule pileux lors de la phase anagène visualisée au microscope.
   Légendes :
   (A) est une série de clichés de la gaine épithéliale externe grossie 40 fois, (B) est une série de clichés de la gaine épithéliale externe (centrés sur la tige) grossie 20 fois et (C) est une série de clichés du bulbe grossie 20 fois.
      (1) représente un cheveu très foncé, (2) un cheveu modérément pigmenté, (3) à (5) des cheveux de différentes nuances de gris et (6) un cheveu blanc.
**Figure 2** **:** Ces photographies permettent de visualiser l'expression de TRP-2 dans les mélanocytes de l'épiderme et du cheveu (gaine épithéliale externe et bulbe pileux). Etude immunohistologique analysée en microscopie confocale laser.
**Figure 3** **:** ces photographies représentent les résultats obtenus après la mise en oeuvre des essais de Western Blot décrits à l'exemple 2B.
**Figure 4** **:** cette photographie représente un Western blot montrant l'effet inducteur de l'expression de TRP2 de la forskoline (Fk) par rapport à un contrôle.

### Exemple 1 - révélation immunohistochimique des mélanocytes dans les follicules pileux à différents stades de blanchissement, par marquage de la protéine pMel-17.

Plus de 120 follicules pileux isolés à partir de biopsies issues de 8 donneurs âgés de 49 à 71 ans ont été étudiés.

### A - Protocole d'isolement des follicules pileux entiers (Commo S and Bernard BA Pigment Cell Res 2000 ; 13 :253-259)

Des fragments de biopsie sont incubés dans la dispase (2,4 U/ml, Boehringer Mannheim, D) la nuit à +04°C. Les cheveux sont ensuite isolés à l'aide de pinces sous binoculaires.

### B - Protocole d'immunomarquage sur follicules pileux entiers (Commo S and Bernard BA Pigment Cell Res 2000 ; 13 :253-259)

Les cheveux entiers sont fixés dans l'éthanol à -20°C pendant 10 minutes. Chaque étape des procédures de fixation et de marquage est suivie de lavages au tampon phosphate (pH 7,4 (PBS))-Tween20 0,05%. Sauf précision, toutes les étapes se font à température ambiante. Les péroxydases endogènes de l'échantillon sont neutralisées en incubant l'échantillon dans une solution de péroxyde d'hydrogène à 0,1% pendant 10 minutes. Pour bloquer les sites de fixation non spécifiques, l'échantillon est incubé avec du lait écrémé 1%, 15 minutes. L'anticorps (Ac) primaire NK1-beteb reconnaissant spécifiquement la protéine pMel-17 (Monosan, Paris, F) est dilué au 1/40 dans du PBS - Tween 0,05%, contenant 10 % de sérum normal (X0907, DAKO, Trappes, F). L'Ac primaire est incubé 18 heures sur les cheveux à +04°C. L'Ac secondaire couplé à la biotine (E-433, DAKO, Trappes, F) est dilué au 1/400 et incubé 30 minutes. Le cheveu est ensuite incubé en présence de streptavidine-biotine-péroxydase (K-0377, DAKO, Trappes, F), et finalement l'immunomarquage est révélé en présence de 3-amino-9-éthylcarbazole (AEC) (AEC kit-101, Sigma, Saint Quentin Fallavier, F.).

En comparant les clichés (B1) à (B5) de la figure 1, on constate que la diminution de pigmentation du cheveu est associée à une diminution de mélanine dans le bulbe et à une diminution de mélanocytes dans le bulbe (voir C1 à C5). Le cheveu blanc dont la tige est dépourvue de mélanine (B6) ne contient pas de mélanocyte dans le bulbe (C6). Les cheveux gris et blanc contiennent une quantité variable de mélanocytes dans la partie haute de la gaine épithéliale externe, cette quantité pouvant même être nulle dans le cas du cheveu blanc (A3 à 6) à la différence des cheveux pigmentés (A1 et 2).

### Exemple 2 - mise en évidence de l'expression différentielle de la DOPAchrome tautomérase dans les mélanocytes de follicules pileux et de l'épiderme chez l'individu caucasien.

### A - Etude immunohistologique analysée en microscopie confocale laser

### A.1 - Obtention de coupes congelées de follicule pileux (Commo S and Bernard BA Pigment Cell Res 2000 ;13 :253-259)

Un fragment de biopsie de scalp contenant des follicules pileux est inclus dans du tissue-Tek-OCT (Miles, Naperville, IL, USA) et ensuite congelé sur de la carbo-glace. La biopsie congelée est ensuite coupée (7 µm) à l'aide d'une cryostat (CM3050, Leica, Rueil-Malmaison, F).

### A.2 - Protocole d'isolement des follicules pileux entiers et des lambeaux épithéliaux de peau (Commo S and Bernard BA Pigment Cell Res 2000 ;13 :253-259)

Des fragments de biopsie sont incubés dans la dispase (2,4 U/ml, Boehringer Mannheim, D) la nuit à +04°C. Le compartiment épithélial est séparé du derme à l'aide de pinces sous binoculaire. Les structures épithéliales sont ensuite micro-disséquées pour séparer les follicules pileux et l'épiderme, puis triées.

### A.3 - Protocole d'immunomarquage sur follicule pileux entier, lambeau de peau et coupe congelée

Les cheveux entiers, les lambeaux épithéliaux de peau et les coupes congelées sont fixés dans l'éthanol à -20°C pendant 10 minutes. Chaque étape des procédures de fixation et de marquage est suivie de lavages au tampon phosphate (pH 7,4 (PBS))-Tween20 0,05%. Sauf précision, toutes les étapes se font à température ambiante. Les péroxydases endogènes de l'échantillon sont neutralisées en incubant l'échantillon dans une solution de péroxyde d'hydrogène à 0,1% pendant 10 minutes. Pour bloquer les sites de fixation non spécifiques, l'échantillon est incubé avec du lait écrémé 1%, 15 minutes. Les anticorps (Ac) primaires sont dilués dans du PBS - Tween 0,05%, contenant 10 % de sérum normal (X0907, DAKO, Trappes, F). Les Ac primaires NK1-beteb reconnaissant spécifiquement la protéine pMel-17 (1/40, Monosan, Paris, F), et αPEP8h (1/2000, Dr VJ.Hearing, NIH, Bethesda, MD, USA) reconnaissant spécifiquement la protéine TRP2 humaine (Virador et al. 2001) sont incubés simultanément 18 heures à +04°C sur les cheveux entiers et les lambeaux épithéliaux de peau, et 30 minutes à température ambiante sur les coupes congelées. L'Ac secondaire de chèvre dirigé contre les immunoglobulines (Ig) G2b couplé à la Cy3 (M32410, TEBU, le Perray en Yveline, F) est dilué au 1/80, et l'Ac secondaire dirigé contre les Ig couplé à la Cy5 (111-175-144, Jackson Immunoresearch Lab. Inc. West Grove, PA, USA) est dilué au 1/500 sont incubés simultanément 30 minutes sur les échantillons. Les immunomarquages sont analysés en microscopie confocale laser (LSM510, Carl Zeiss, Oberkochen, D).

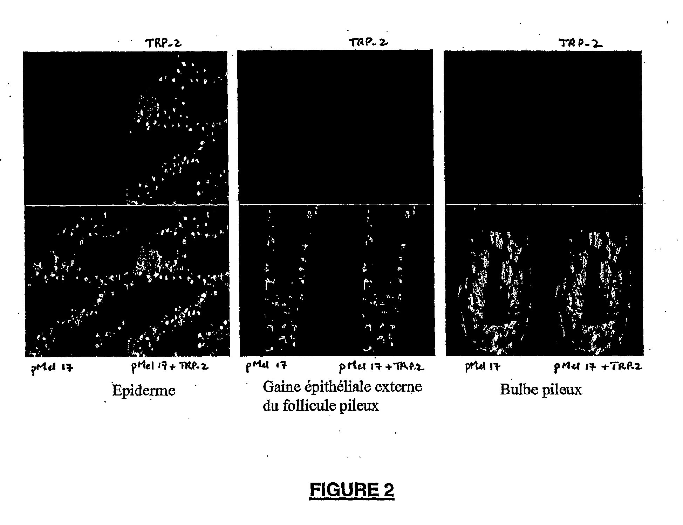

Conclusion des observations : sur la figure 2, on constate la présence de TRP-2 dans les mélanocytes de l'épiderme, en revanche, cette enzyme n'est exprimée ni dans les mélanocytes de la gaine épithéliale du follicule pileux, ni dans les mélanocytes du bulbe pileux.

### B - étude biochimique par analyse en Western Blot

### B.1 - Protocole d'extraction de protéine de follicules pileux humain et de mélanocytes (Commo S et al. Differentiation 2000 ;66 :157-164)

- Extraction protéique à partir de follicules pileux : les follicules pileux sont isolés après traitement de biopsies de scalp à la dispase (2,4 U/ml, Boehringer Mannheim, D) la nuit à +04°C. Après isolement, les follicules pileux sont micro-disséqués pour isoler la partie du bulbe pileux. 80 bulbes pileux ainsi isolés sont placés dans un tampon de lyse approprié pour extraction protéique et analyse en *Western Blot.*
- Extraction protéique à partir de culture de mélanocytes : Les mélanocytes cultivés en milieu M2 (PromoCell, Heidelberg, D) sont lysés avec un même tampon de lyse approprié pour extraction protéique et analyse en *Western Blot.*

Le *Western blot* (voir protocole dans Maniatis *et al*.) est réalisé avec les anticorps suivants : αPEP8h, anticorps polyclonal spécifique de la TRP-2 humaine donné par Dr VJ Hearing (NIH, Bethesda, USA), et T311, anticorps monoclonal spécifique de la tyrosinase humaine (Novocastra, New Castle, UK).

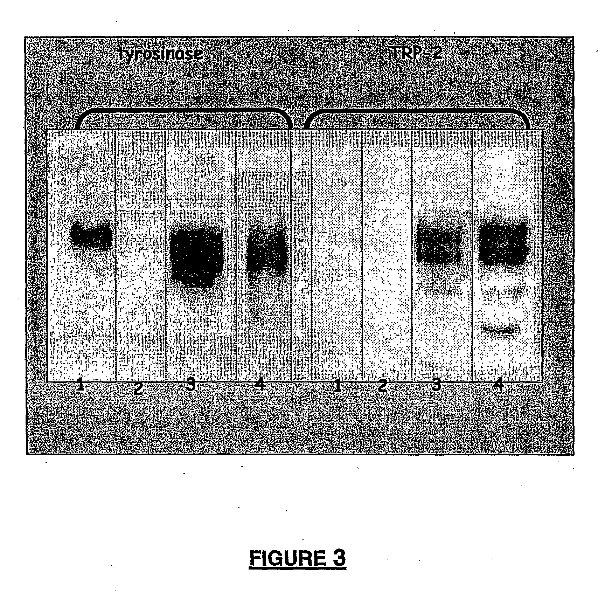

### Observations et commentaires de la figure 3 :

On observe que la tyrosinase est détectée dans les extraits de bulbe pileux. L'enzyme n'est pas détectée dans les extraits de gaine épithéliale externe. L'expression de la tyrosinase est régulée. Cette enzyme n'est pas ou peu exprimée dans les mélanocytes inactifs (ne produisant pas de mélanine), c'est le cas des mélanocytes contenus dans le scalp inter folliculaire d'individu caucasien.

Par ailleurs, la DOPAchrome tautomérase (TRP-2) n'est détectée ni dans les extraits de bulbe ni dans les extraits de gaine épithéliale externe. L'expression de la TRP-2 ne suit pas celle de la tyrosinase et l'induction de la mélanogénèse, elle n'est pas exprimée dans les mélanocytes actifs des bulbes pileux.

### Exemple 3 : Mise en évidence de l'effet inducteur de la forskoline sur l'expression de la DOPAchrome tautomérase (TRP2)

Dans une première étape (a), les mélanocytes sont ensemencés à J0 avec du milieu M2 (PromoCell, Heidelberg, D). Après un temps nécessaire à l'adhérence des cellules compris entre 2 et 18 heures, le milieu est remplacé par un milieu dans lequel les mélanocytes n'expriment peu ou pas la DOPAchrome tautomerase (expression de TRP-2 basale) ou bien expriment une DOPAchrome tautomerase inactive : DMEM :F12 (Gibco BRL - 42400-044), Ultroser G (Gibco BRL - 15950-017) 0,5%, PC-1 (BioWhittaker 344022) 0,5%, bFGF (Pepro Tech Inc 100-18B) 5 ng/ml, héparine (Sigma H-3149) 75 ng/ml, 1% antibiotiques, 1% glutamine. Les cellules sont maintenues dans ce milieu de culture le temps compris entre 12 et 72 heures nécessaire à la diminution de l'expression de TRP-2.

Dans une étape (b), la forskoline (20µM) est ajoutée au milieu de culture ; les mélanocytes sont incubés dans ce milieu pendant 24h (étape c).

La révélation du taux de TRP2 (étape d) se fait par la méthode classique du Western blot, en présence d'un anticorps αPEP8h reconnaissant spécifiquement la protéine TRP2 humaine (Virador et al. 2001).

La vimentine (protéine du cytosquelette des mélanocytes) est utilisée pour assurer que la charge protéique est équivalente dans les différents essais.

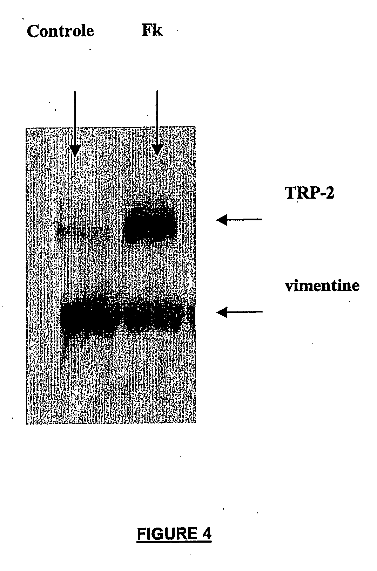

Les résultats présentés à la Figure 4 montrent que la forskoline est capable d'induire l'expression de la DOPAchrome tautomérase (TRP2), par rapport au contrôle.

### Exemple 4 - Compositions

### - lotion capillaire

| | |
|---|---|
| Inducteur de la DOPAchrome tautomérase | 0,5 g |
| Propylène glycol | 20 g |
| Ethanol 95° | 30 g |
| Eau qsp | 100 g |

Cette lotion est appliquée quotidiennement sur les zones à traiter et de préférence sur l'ensemble du cuir chevelu pendant au moins 10 jours et préférentiellement 1 à 2 mois. On constate alors une diminution de l'apparition des cheveux blancs ou gris et une repigmentation des cheveux gris.

### - shampooing traitant

| | |
|---|---|
| Inducteur de la DOPAchrome tautomérase | 1,5 g |
| Polyglycéryl 3-hydroxylarylether | 26 g |
| Hydroxy propyl cellulose vendue sous la dénomination de Klucell G par la société Hercules | 2 g |
| Conservateurs | ps |
| Ethanol 95° | 50 g |
| Eau qsp | 100 g |

Ce shampooing est utilisé à chaque lavage avec un temps de pose d'environ d'une minute. Un usage prolongé, de l'ordre de deux mois, conduit à la repigmentation progressive des cheveux gris.

Ce shampooing peut également être utilisé à titre préventif afin de retardé le blanchiment des cheveux.

### - Gel traitant

| | |
|---|---|
| Inducteur de la DOPAchrome tautomérase | 0,75 g |
| Huiles essentielles d'Eucalyptus | 1 g |
| Econozole | 0,2 g |
| Lauryl polyglyceryl 6 cetearyl glycoether | 1,9 g |
| Conservateurs | qs |
| Carbopol 934P vendu par la société BF Goodrich Corporation | 0,3 g |
| Agent de neutralisation | qs pH 7 |
| Eau qsp | 100 g |

Ce gel est appliqué sur les zones à traiter deux fois par jour (matin et soir) avec un massage terminal. Après trois mois d'application, on observe une repigmentation des poils ou cheveux de la zone traitée.

## Revendications

1. Utilisation cosmétique, non-thérapeutique, d'un agent inducteur de l'expression de la DOPAchrome tautomérase, en tant qu'agent protecteur des mélanocytes du follicule pileux, ledit agent étant le kaempférol encapsulé.

2. Utilisation selon la revendication 1, **caractérisée en ce que** l'agent inducteur de l'expression de la DOPAchrome tautomérase est destiné à lutter contre la disparition des mélanocytes du follicule pileux en maintenant et/ou en régénérant la population des mélanocytes actifs du bulbe et des mélanocytes quiescents de la région supérieure du follicule pileux.

3. Utilisation selon l'une des revendications 1 ou 2, **caractérisée en ce que** l'agent inducteur de l'expression de la DOPAchrome tautomérase est destiné à favoriser le renouvellement cyclique de l'unité folliculaire de pigmentation.

4. Utilisation selon l'une des revendications 1 à 3, **caractérisée en ce que** l'agent inducteur de l'expression de la DOPAchrome tautomérase est destiné à prévenir et/ou limiter et/ou arrêter le développement de la canitie.

5. Utilisation selon l'une des revendications 1 à 3, **caractérisée en ce que** l'agent inducteur de l'expression de la DOPAchrome tautomérase est destiné à maintenir la pigmentation naturelle des cheveux et/ou des poils gris.

6. Utilisation non-thérapeutique d'une composition cosmétique comprenant dans un milieu cosmétiquement acceptable au moins un agent inducteur de l'expression de DOPAchrome tautomérase pour protéger les mélanocytes du follicule pileux, ledit agent étant le kaempférol encapsulé.

7. Utilisation selon la revendication 6, **caractérisée en ce qu'**elle comprend une quantité d'agent inducteur de l'expression de la DOPAchrome tautomerase comprise entre 0,001 et 10 % en poids par volume, préférentiellement entre 0,01 et 5% en poids par volume et encore plus préférentiellement entre 0,1 et 1 % en poids par volume.

8. Utilisation selon l'une quelconque des revendications 6 ou 7, **caractérisée en ce que** la composition est adaptée à une composition orale.

9. Composition selon l'une quelconque des revendications 6 ou 7, **caractérisée en ce que** la composition est adaptée à une application topique sur le cuir chevelu et/ou sur les zones de la peau recouverte de poils.

10. Composition selon la revendication 9, **caractérisée en ce que** la composition est une crème ou un gel coiffant, une lotion capillaire, en particulier une lotion de mise en plis ou une lotion traitante ou une lotion restructurante pour les cheveux, une composition de teinture, un shampoing ou un après-shampoing.

11. Utilisation selon l'une quelconque des revendications 6 à 10, **caractérisée en ce que** ledit au moins un agent inducteur de l'expression de DOPAchrome tautomérase est encapsulé dans un enrobage tel que les microsphères, des nanosphères, des oléosomes ou des monocapsules.

12. Utilisation selon la revendication 11, **caractérisée en ce que** l'enrobage dans lequel l'agent inducteur de l'expression de la DOPAchrome tautomerase est encapsulé à un diamètre inférieur ou égale à 10 µm.

13. Utilisation selon la revendication 12, **caractérisée en ce que** l'agent inducteur de l'expression de DOPAchrome tautomérase est associé à un autre actif choisi parmi les agents de lutte des états desquamatifs du cuir chevelu, des agents favorisant la repousse des cheveux, des extraits végétaux à activité propigmentante.

## Patentansprüche

1. Nichttherapeutische kosmetische Verwendung eines Wirkstoffs, der die Expression der DOPAchromtautomerase induziert, als Schutzmittel für Haarfollikelmelanozyten, wobei es sich bei dem Wirkstoff um verkapseltes Kämpferol handelt.

2. Verwendung nach Anspruch 1, **dadurch gekennzeichnet, dass** der Wirkstoff, der die Expression der DOPAchromtautomerase induziert, für die Bekämpfung des Verschwindens der Haarfollikelmelanozyten bestimmt ist, wobei die Population der aktiven Melanozyten der Haarzwiebel und der ruhenden Melanozyten der Zone oberhalb des Haarfollikels aufrechterhalten und/oder regeneriert wird.

3. Verwendung nach einem der Ansprüche 1 oder 2, **dadurch gekennzeichnet, dass** der Wirkstoff, der die Expression der DOPAchromtautomerase induziert, zur Förderung der zyklischen Erneuerung der Pigmentierungsfollikeleinheit bestimmt ist.

4. Verwendung nach einem der Ansprüche 1 bis 3, **dadurch gekennzeichnet, dass** der Wirkstoff, der die Expression der DOPAchromtautomerase induziert, zum Vorbeugen und/oder Einschränken und/oder Stoppen des Ergrauens bestimmt ist.

5. Verwendung nach einem der Ansprüche 1 bis 3, **dadurch gekennzeichnet, dass** der Wirkstoff, der die Expression der DOPAchromtautomerase induziert, zur Aufrechterhaltung der natürlichen Pigmentierung von grauem Haar bestimmt ist.

6. Nichttherapeutische Verwendung einer Kosmetikzusammensetzung, die mindestens einen Wirkstoff, der die Expression der DOPAchromtautomerase induziert, in einem kosmetisch unbedenklichen Medium umfasst, um die Melanozyten der Haarfollikel zu schützen, wobei es sich bei dem Wirkstoff um verkapseltes Kämpferol handelt.

7. Verwendung nach Anspruch 6, **dadurch gekennzeichnet, dass** die Zusammensetzung den Wirkstoff, der die Expression der DOPAchromtautomerase induziert, in einer Menge zwischen 0,001 und 10 Gew.-% in Bezug auf das Volumen, vorzugsweise zwischen 0,01 und 5 Gew.-% in Bezug auf das Volumen und noch stärker bevorzugt zwischen 0,1 und 1 Gew.-% in Bezug auf das Volumen umfasst.

8. Verwendung nach einem der Ansprüche 6 oder 7, **dadurch gekennzeichnet, dass** die Zusammensetzung an eine Oralzusammensetzung angepasst ist.

9. Zusammensetzung nach einem der Ansprüche 6 oder 7, **dadurch gekennzeichnet, dass** die Zusammensetzung an eine topische Anwendung auf die Kopfhaut und/oder die von Haar bedeckten Regionen der Haut angepasst ist.

10. Zusammensetzung nach Anspruch 9, **dadurch gekennzeichnet, dass** es sich bei der Zusammensetzung um eine Frisiercreme bzw. ein Frisiergel, eine Haarlotion, insbesondere eine Wasserwellenlotion oder eine behandelnde Lotion oder eine restrukturierende Lotion für das Haar, eine Färbezusammensetzung, ein Shampoo oder eine Pflegespülung handelt.

11. Verwendung nach einem der Ansprüche 6 bis 10, **dadurch gekennzeichnet, dass** der Wirkstoff, der die Expression der DOPAchromtautomerase induziert, in einer Umhüllung wie Mikrosphären, Nanosphären, Oleosomen oder Monokapseln verkapselt ist.

12. Verwendung nach Anspruch 11, **dadurch gekennzeichnet, dass** die Umhüllung, in der der Wirkstoff, der die Expression der DOPAchromtautomerase induziert, verkapselt ist, einen Durchmesser von 10 µm oder weniger aufweist.

13. Verwendung nach Anspruch 12, **dadurch gekennzeichnet, dass** der Wirkstoff, der die Expression der DOPAchromtautomerase induziert, mit einem weiteren Wirkstoff, ausgewählt aus den Mitteln zur Bekämpfung von schuppigen Zuständen der Kopfhaut, Mitteln, die den Neuzuwachs von Haar fördern, und Pflanzenextrakten mit pigmentfördernder Wirksamkeit, kombiniert ist.

## Claims

1. Non-therapeutic cosmetic use of an agent inducing the expression of DOPAchrome tautomerase, as agent protecting the melanocytes of the hair follicle, said agent being the encapsulated kaempferol.

2. Use according to Claim 1, **characterized in that** the agent inducing the expression of DOPAchrome tautomerase is intended to combat the disappearance of the melanocytes of the hair follicle by maintaining and/or by regenerating the population of active melanocytes of the bulb and of the quiescent melanocytes of the top region of the hair follicle.

3. Use according to either of Claims 1 and 2, **characterized in that** the agent inducing the expression of DOPAchrome tautomerase is intended to promote the cyclic renewal of the follicular pigmentation unit.

4. Use according to one of Claims 1 to 3, **characterized in that** the agent inducing the expression of DOPAchrome tautomerase is intended to prevent and/or limit and/or arrest the development of canities.

5. Use according to one of Claims 1 to 3, **characterized in that** the agent inducing the expression of DOPAchrome tautomerase is intended to maintain the natural pigmentation of gray head hair and/or body hair.

6. Non-therapeutic use of a cosmetic composition, comprising, in a cosmetically acceptable medium, at least one agent inducing the expression of DOPAchrome tautomerase for protecting the melanocytes of the hair follicle, said agent being the encapsulated kaempferol.

7. Use according to Claim 6, **characterized in that** the composition comprises a quantity of agent inducing the expression of DOPAchrome tautomerase of between 0.001 and 10% by weight per volume, preferably between 0.01 and 5% by weight per volume and still more preferably between 0.1 and 1% by weight per volume.

8. Use according to either one of Claims 6 and 7, **characterized in that** the composition is suitable for an oral composition.

9. Composition according to either one of Claims 6 and 7, **characterized in that** the composition is suitable for topical administration to the scalp and/or to the areas of the skin covered with body hair.

10. Composition according to Claim 9, **characterized in that** the composition is a hair styling cream or gel, a hair lotion, in particular a hair setting lotion or a treatment lotion or a restructuring lotion for the hair, a dye composition, a shampoo or a conditioner.

11. Use according to any one of Claims 6 to 10, **characterized in that** said at least one agent inducing the expression of DOPAchrome tautomerase is encapsulated in a coating such as microspheres, nanospheres, oleosomes or monocapsules.

12. Use according to Claim 11, **characterized in that** the coating in which the agent inducing the expression of DOPAchrome tautomerase is encapsulated has a diameter of less than or equal to 10 µm.

13. Use according to Claim 12, **characterized in that** the agent inducing the expression of DOPAchrome tautomerase is combined with another active agent chosen from agents for combating desquamative states of the scalp, agents promoting hair regrowth, plant extracts with propigmenting activity.
